# EUROPEAN PATENT SPECIFICATION

(11) **EP 1 041 919 B1**
(45) Date of publication and mention of the grant of the patent: **15.03.2006**
(21) Application number: 98960078.8
(22) Date of filing: 22.12.1998
(51) Int. Cl.: A61B 1/012, A61M 31/00

(54) **SYSTEM FOR IN VIVO DELIVERY OF AUTONOMOUS CAPSULE**
ANORDNUNG ZUR IN VIVO VERABREICHUNG VON AUTONOMEN KAPSELN
SYSTEME SERVANT A ADMINISTRER IN VIVO UNE CAPSULE AUTONOME

(30) Priority: 22.12.1997 IL 12271697
(43) Date of publication of application: 11.10.2000
(73) Proprietor: Given Imaging Ltd., 20692 Yokneam Ilite (IL)
(72) Inventor: Iddan, Gavriel, J., 34602 Haifa (IL); Meron, Gavriel, 49556 Petach Tikva (IL)
(74) Representative: Gray, John James
(86) International application number: PCT/IL1998/000621
(87) International publication number: WO 1999/032028

(56) References cited:
- US-A- 4 027 510
- US-A- 4 198 960
- US-A- 5 373 840

## Description

The present invention concerns a delivery system for autonomous capsules used in internal imaging of the gastro-intestinal tract.

Endoscopic inspection is a common practice in the medical diagnosis of gastro-intestinal (G.I.) diseases. According to such a method, the video camera used for identifying observable irregularities of the internal lining of the G.I. tract is installed within an endoscope, with progressive scenes observed by pushing the endoscope inside the tract. The endoscope is a tubular device typically containing an image collecting device, a light source and optionally a remotely controlled mechanical appliance for sampling tissue and for manipulating the endoscope tip.

A device such as the tissue sampler, which is a claw- like utility for picking out tissue parts for purposes such as biopsies, is generally manipulated by a cable or a rod. For that purpose, endoscopes often comprises a bore for housing such longitudinal mechanical power drivers.

Because the movement of the endoscope head along the G.I. tract is brought about by a pushing action, there are effects associated with the application of force which become especially adverse as bends in the G.I. tract impede the movement of the endoscope. The G.I. tract walls at the bends become susceptible to perforation, making the internal in vivo application of probes, notably endoscopes, limited in use to non-convoluted regions of the G.l. tract.

An *in-vivo* autonomous capsule, such as the one described in US Patent 5,604,531, moves along the G.I. tract by virtue of the natural squeezing action of the tract's walls, thus overcoming the risk associated with the pushing. Another advantage arising from the employment of such an autonomous device, is that it offers a much more convenient method of administering a sensor to the Gl tract, overcoming the cumbersome aspects of connecting the intestines of the patient to external appliances. Thus, data signals, typically electronic, of the gastro-intestinal tract are obtained without physical connections being made to an energy source or a physical information download link. Autonomous capsules are potentially convenient and useful tools for acquiring information of the inner lining of the G.I. tract, being especially beneficial for searching the small intestines which are highly convoluted. Other autonomous capsule types are used in medicine, such as pH measuring, motility measuring, pressure measuring, and those used for internal administration of medicaments.

It is an object of the present invention to provide a device for inserting an autonomous capsule in the G.I. tract, in a -manner that the capsule begins its autonomous journey in the small intestines, while obviating the need to travel along the upper part of the G.I. tract.

In the invention as defined in Claim 1 of the appended claims, a device is provided for delivering autonomous capsules into the G.I. tract. Such a device includes an endoscope having a longitudinal axis and a ring clamp for releasably holding the capsule whereby its longitudinal axis lies along the same axis as the longitudinal axis of the endoscope. The clamp is held in the front of the endoscope by at least one retractable support. A forward looking imaging unit may also be situated at the front end of the endoscope.

It is noted that various devices are known for performing different operations within the body. The following are three examples.

US 4198960 which teaches flexible cables passing through a ring with holes to form a cage. The flexible cables may be manually retracted in-vivo, to trap in vivo objects such as stones within the cage.

US 4027510 discloses an endoscope provided with a tweezers shaped clamp for clamping fallopian tubes.

US 5373840 teaches a tweezers clamp and a ring shaped separating element to separate a blood vessel from surrounding tissue, for harvesting the blood vessel for use in another part of the body.

### BRIEF DESCRIPTION OF THE DRAWINGS

The present invention will be understood and appreciated more fully from the following detailed description taken in conjunction with the appended drawings in which:
Fig. 1 is a schematic illustration of a delivery system for inserting autonomous capsules for data collecting, in the G.I. tract;
Fig. 2 is a schematic illustration of a delivery system as in Fig. 1 wherein the direction of pulling the clamp supports is shown; and
Fig. 3 is a schematic illustration of a detached capsule with fully retracted supports, and the fields of view of both imaging systems is marked in arrows.

### DETAILED DESCRIPTION OF THE INVENTION

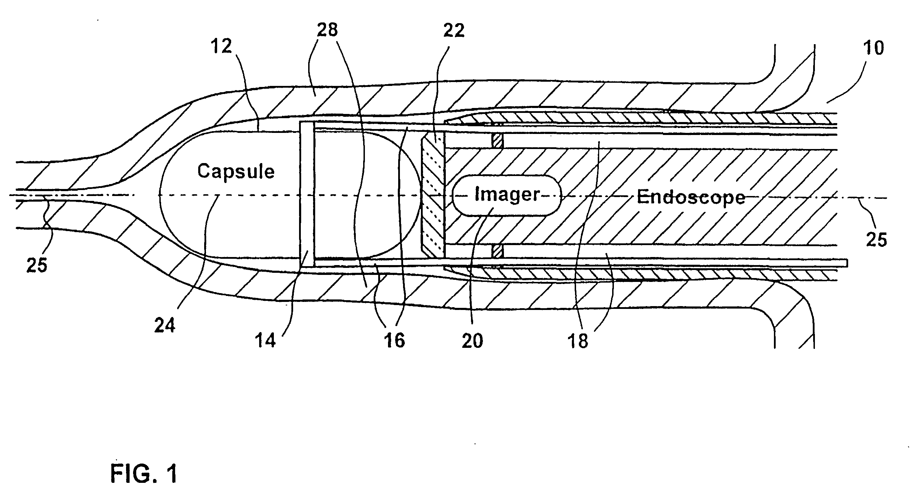

Reference is now made to Fig. 1, which schematically shows a modified endoscope 10 engaging a capsule 12, constructed and operative in accordance with a preferred embodiment of the present invention. The endoscope device 10, shown inserted tightly within the walls of a G.I. tract 28, comprises a ring clamp 14 with retractable supports 16. The endoscope 10 also comprises a camera (imager) 20, for taking images through an optical window 22.

The capsule 12 is attached to the front of the endoscope with its longitudinal axis 24 parallel (and in line with) to the longitudinal axis 25 of the endoscope. The capsule 12, which abuts window 22, is held in place by the ring clamp 14, which is itself supported by the pair of retractable supports 16. Retractable supports 16 are movable within a bore 18, along the entire length of the endoscope 10.

The modified endoscope 10 of the invention can insert an autonomous capsule 12 in a target location within the G.I. tract 28 in a manually controlled fashion, thereby achieving several goals. In particular, endoscope 10 can be used to expeditiously insert the autonomous capsule 12 in a desired location, thus reducing the time required for the autonomous capsule to reach its target. As a result, the autonomous capsule has more time in which to collect data. The modified endoscope can be used in the non-convoluted terminal of the G.I. tract.

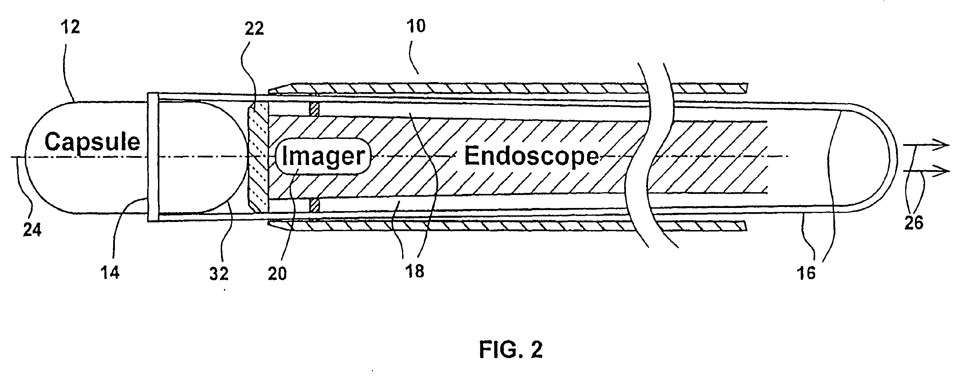

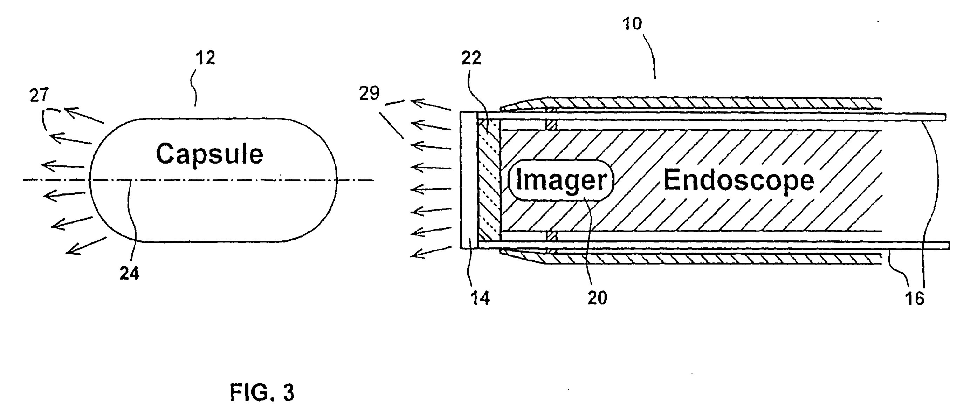

Fig. 2 shows the device of Fig. 1 with the ends of the clamp supports 16 shown protruding outside of the patient's body. The arrows 26 indicate the direction of pull needed to bring about the retraction for disengaging the capsule 12. The capsule, being substantially cylindrical, is held snugly by the ring clamp 14. When the clamp's supports 16 are retracted within bore 18, the clamp 14 slides along the smooth surface of the capsule, and eventually loosens its grip on the capsule 12. Thus, the capsule 12 is deposited in position as soon as full retraction of the clamp 14 has taken place. Fig. 2 illustrates the capsule 12 retracted to a stage in which the capsule 12 abuts against the window 22 of the endoscope 10. The window 22 therefore blocks the capsule's further retraction movement, thereby facilitating the sliding of the ring 14 on the capsule's surface. Disengagement of the capsule takes place only as the clamp 14 has slipped by the back end (referenced 32) of the capsule 12, due to the pulling of the supports 16 manually in the direction indicated by arrows 26 away from the capsule 12. This particular situation is shown in Fig. 3, which also shows the capsule 12 detached from the endoscope 10.

An autonomous capsule of an imaging type, such as described in US Patent No: 5,604,531, can be used to verify its own place of insertion in the G.l. tract as it is pushed along. Once it is deposited, it can continue to acquire images autonomously. Fig. 3, shows the viewing range (arrows 27) of the detached capsule 12, as well as the viewing range (arrows 29) of the imager 20 in the endoscope. The endoscope becomes operative as a camera once the capsule 12 has detached.

It will be appreciated that the present invention is not limited by what has been described hereinabove and that numerous modifications, all of which fall within the scope of the present invention, exist. For example, the number of supports of the clamp can be other than described.

Rather the scope of the invention is defined by the claims which follow:

## Claims

1. A device for delivering an autonomous capsule (12) having a first longitudinal axis (24), into the G.I. tract (28), the device comprising:
an endoscope (10) having a second longitudinal axis (25);
a ring clamp (14) for releasably holding said capsule; and
at least one retractable support (16) for retaining said ring clamp at the front end of said endoscope, said at least one support being movable within said endoscope.

2. A device according to claim 1 and further comprising an imaging unit (10) situated at the front end of said endoscope.

3. A device according to claim 1 or 2 wherein said clamp is arranged to hold said capsule so that said first longitudinal axis lies on the same axis as said second longitudinal axis.

## Revendications

1. Dispositif de délivrance d'une capsule autonome (12) présentant un premier axe longitudinal (24) dans la voie gastro-intestinale (28), comprenant :
un endoscope (10) présentant un second axe longitudinal (25) ;
une pince annulaire (14) pour maintenir de manière détachable ladite capsule et
au moins un support rétractable (16) pour retenir ladite pince annulaire à l'extrémité frontale dudit endoscope, ledit au moins un support étant mobile à l'intérieur dudit endoscope.

2. Dispositif selon la revendication 1, comprenant en outre une unité d'imagerie (10) située à l'extrémité frontale dudit endoscope.

3. Dispositif selon la revendication 1 ou 2, dans lequel ladite pince est conçue pour maintenir ladite capsule de manière à ce que ledit premier axe longitudinal se trouve sur le même axe que ledit second axe longitudinal.

## Patentansprüche

1. Gerät, um eine autonome Kapsel (12) mit einer ersten longitudinalen Achse (24) in den Magen-Darm-Trakt (28) zu liefern, wobei das Gerät ein
Endoskop (10) mit einer zweiten longitudinalen Achse (25):
eine Ringklemme (14) um die Kapsel auslösbar festzuhalten: und
zumindest eine zurückziehbare Halterung (16) um die Ringklemme am vorderen Ende des Endoskops zu halten und wobei zumindest eine Halterung beweglich innerhalb des Endoskops ist, umfasst.

2. Gerät nach Anspruch 1 dass außerdem noch eine Bildaufnahmeeinheit (10) am vorderen Ende des Endoskops umfasst.

3. Gerät nach Anspruch 1 oder Anspruch 2 wobei die Klemme so arrangiert ist, um die Kapsel derart zu halten, dass die erste longitudinale Achse auf der selben Achse liegt als die zweite longitudinale Achse.
